# EUROPÄISCHE PATENTSCHRIFT

(11) **EP 2 845 615 B1**
(45) Veröffentlichungstag und Bekanntmachung des Hinweises auf die Patenterteilung: **23.06.2021**
(21) Anmeldenummer: 14184304.5
(22) Anmeldetag: 10.09.2014
(51) Int. Cl.: A61M 13/00, A61M 11/00, A61M 25/10, A61M 31/00, A61M 15/02, A61M 16/04, A61M 25/01, A61B 1/05, A61B 10/04, A61B 17/00, A61B 17/22, A61B 17/32, A61B 17/34

(54) **THERAPIEVORRICHTUNG ZUR GABE VON AEROSOL**
THERAPY DEVICE FOR ADMINISTRATION OF AEROSOL
DISPOSITIF THÉRAPEUTIQUE POUR L'ADMINISTRATION D'UN AÉROSOL

(30) Priorität: 10.09.2013 DE 102013109896; 10.09.2013 DE 202013104123 U
(43) Veröffentlichungstag der Anmeldung: 11.03.2015
(73) Patentinhaber: Hetzel, Alexander, 78667 Villingendorf (DE); Reymond, Marc, A., 44626 Herne (DE)
(72) Erfinder: Hetzel, Alexander, 78667 Villingendorf (DE); Reymond, Marc, A., 44626 Herne (DE)
(74) Vertreter: mepat Patentanwälte

(56) Entgegenhaltungen:
- WO-A1-2008/063179
- WO-A1-2012/163346
- WO-A1-2013/090730
- WO-A2-02/36190
- WO-A2-2007/103809
- DE-T2- 69 001 994
- DE-T2- 69 522 744
- JP-A- 2003 079 730
- US-A- 3 709 214
- US-A1- 2003 028 227
- US-A1- 2003 159 696
- US-A1- 2004 221 854
- US-A1- 2005 005 936
- US-A1- 2007 083 158
- US-A1- 2009 023 997
- US-A1- 2009 124 999
- US-A1- 2011 030 680
- US-A1- 2012 209 166
- US-A1- 2013 123 682

## Beschreibung

Die Erfindung betrifft eine Vorrichtung der Medizintechnik und insbesondere eine Vorrichtung durch die ein Verfahren zur örtlichen Abtötung von Tumoren in Körperhöhlen angewandt werden kann. Unter Körperhöhlen werden im Sinne der Erfindung auch Organe, insbesondere Hohlorgane von Mensch und Tier verstanden, insbesondere Organe wie Speiseröhre, Magen, Darm, Luftröhre, Gebärmutter, Harnröhre, Harnblase und Eileiter.

Aus der WO 2012/163346 A1 ist bereits eine Vorrichtung zum Ausbringen einer Substanz in einer Körperhöhle bekannt. Bei der Substanz kann es sich beispielsweise um Zytostatika wie Doxorubicin, Cisplatin oder andere Chemotherapeutika handeln. Insbesondere ist nach der WO 2012/163346 A1 vorgesehen, dass die unter hohem Druck geförderte Substanz an einer Nadeldüse nebelartig in ein Aerosol zerstäubt wird. Der gewonnene Aerosolnebel kann sich an die gesamte vom Nebel erreichbare Oberfläche der Körperhöhle anlegen und die Substanz dort ihre vorteilhafte Wirkung entfalten. Die Vorrichtung der WO 2012/163346 A1 kann verwendet werden in einem Verfahren zum gerichteten Ausbringen einer Substanz in einem Hohlraum, wie einem Hohlorgan, einer Körperhöhle, insbesondere einem therapeutischen Pneumoperitoneum, mit den Schritten: a. Einführen einer Trokarhülse ein Trokarsystem mit einer Trokarhülse, b. Beaufschlagen der Trokarhülse mit einem Insuffliergas, insbesondere CO₂, c. Durchdringen der Trokarhülse mit einem Düsensystem, welches in seinem Inneren ein Lumen ausbildet, mit einem proximalen Ende und einem distalen Ende, wobei das Düsensystem eine an dem distalen Ende mit dem Lumen befestigte Nadeldüse aufweist und durch die Trokarhülse geführt ist, d. Erzeugen eines Aerosols über die Nadeldüse in dem Hohlraum. Um ein fluiddichtes, geschlossenen System auszubilden, wird in der Vorrichtung der WO 2012/163346 A1 ein Zugang in der Bauchdecke des Patienten mittels einer Schutzmanschette verschlossen, die einerseits an der Hautoberfläche um den Zugang herum fluiddicht angebracht ist und andererseits an der Umfangsfläche des Trokarsystems mit der Trokarhülse fluiddicht anliegt.

Eine weitere Vorrichtung zur Einführung in eine Körperhöhle, zur Insufflation der Körperhöhle mit CO₂ und zur Erzeugung eines therapeutischen Aerosols in der Körperhöhle ist aus der US 2012/0209166 A1 bekannt.

Eine Aufgabe der Erfindung ist es, eine alternative Vorrichtung vorzuschlagen, mittels der eine Substanz lokal begrenzt und hochwirksam innerhalb einer Körperhöhle ausgebracht werden kann. Insbesondere ist eine Aufgabe der Erfindung, die natürlich vorhandenen Zugänge von Organen, insbesondere von Hohlorganen für eine örtlich definierbare Aerosolapplikation innerhalb der Organe zu nutzen und dabei auf resezierende Eingriffe möglichst zu verzichten.

Die Aufgabe kann durch eine Vorrichtung gemäß dem Anspruch 1 gelöst werden. Die Unteransprüche definieren bevorzugte Ausführungsformen der Erfindung.

Es wird dazu eine Vorrichtung geeignet zur Einführung und zur Verwendung innerhalb eines Organs, insbesondere einer Körperhöhle eines Patienten, die Vorrichtung aufweisend eine erste aufweitbare Fluidkammer zur Abdichtung eines ersten Abschnitts der Körperhöhle, eine Aerosolerzeugungsvorrichtung zur Erzeugung eines therapeutischen Aerosols innerhalb eines sich nach dem ersten Abschnitt erstreckenden Behandlungsabschnitts des Organs bzw. der Körperhöhle, beschrieben.

Ferner kann es gewünscht sein, die Wirkung - und damit auch die oftmals verbundene Nebenwirkung - der verwendeten Substanz auf die und nur die Stellen oder Abschnitte der zu behandelnden Körperhöhle zu beschränken, die tatsächlich dieser Wirkung bedürfen. Beispielsweise kann bei einigen Krebsarten diagnostiziert werden, dass sich die zu beobachtenden erkrankten Zellen nicht oder noch nicht die gesamte Körperhöhle betreffen sondern stattdessen ein lokal begrenzter Tumor oder mehrere dicht beieinander liegende Tumore vorhanden sind. Die lokale Beschränkung der Wirkung ist umso mehr erwünscht, je wirksamer d.h. auch toxischer die ausgewählte Substanz ist. Hierzu weist die Vorrichtung in einer weiteren Ausführung der Erfindung eine zweite aufweitbare Fluidkammer zur Abdichtung eines zweiten Abschnitts der Körperhöhle auf, wobei der Behandlungsabschnitt als Mittelababschnitt zwischen dem ersten und dem zweiten Abschnitt vorgesehen ist. Bei der Fluidkammer kann es sich etwa um einen dehnbaren Ballon, einen sogenannten Cuff, bzw. einen abdichtbaren dehnbaren Schlauch aus einem Elastomer handeln. Die Fluidkammer liegt nach Einblasen eines Fluids, etwa der Umgebungsluft, dicht an der jeweiligen Wandung der Körperhöhle an oder dichtet die Körperhöhle ab und kann diese unter Umständen und je nach Wahl des ersten Abschnitts der Körperhöhle dabei auch in geringem Umfang dehnen. Zur besseren Entfaltung der Wirkung des Aerosols beinhaltet die Vorrichtung eine Insuffliervorrichtung zum Insufflieren eines gasförmigen Mediums in den Behandlungsabschnitt der Körperhöhle. Es kann mittels der Insuffliervorrichtung der Behandlungsabschnitt der Körperhöhle mit entsprechendem Druck des gasförmigen Mediums aufgeweitet werden, so dass sich etwaige Falten der Körperhöhlenwandung glätten. Als gasförmiges Medium wird CO₂ verwendet. Als günstig hat sich ein durch die Insuffliervorrichtung aufgebrachter Druck von wenigen Hundertstel bis wenigen Zehntel Millibar herausgestellt. Besonders günstig hat sich erwiesen wenn der Druck zwischen 10 und 15 mbar bei einem Insufflieren von Hohlorganen der Atemwege, insbesondere der Luftröhre, bzw. 12 bis 15 mmHg, also etwa 16 bis 20 mbar bei Insufflieren anderer Hohlorgane beträgt. Die Einleitung und Druckbeaufschlagung des gasförmigen Mediums erfolgt noch vor der Erzeugung des Aerosols.

Nach erfolgter Abdichtung mittels der Fluidkammer und Insufflation kann nun der zur therapeutischen Behandlung vorgesehene Behandlungsabschnitt der Körperhöhle mit Aerosol beaufschlagt werden. Hierzu kann die Aerosolvorrichtung beispielsweise als Düse, insbesondere als Nadeldüse vorgesehen sein, die über eine Zuleitung an eine Pumpe angeschlossen ist.

In einer bevorzugten Ausführungsform ist die erste Fluidkammer gleichzeitig auch die einzige Fluidkammer, die verwendet werden muss. Insbesondere ist vorgesehen, dass diese Fluidkammer im Fall der Verwendung der Vorrichtung innerhalb der Luftröhre schon an einem an sich bekannten Endotrachealtubus oder ähnlichem vorgesehen ist. Der bekannte Endotrachealtubus kann etwa einen leicht gebogenen, etwa 25-30 cm langen Kunststoffschlauch beinhalten, dessen äußerer Durchmesser in etwa dem Kleinfingerdurchmesser des Patienten entspricht. Am mundseitigen Ende kann der Tubus einen Anschlussstutzen für ein Beatmungsgerät oder einen Beatmungsbeutel aufweisen. Bei Verwendung des Aerosols sollte auch in diesem Fall sichergestellt werden, dass kein Aerosol in die Umgebungsluft gelangen kann. Das gegenüberliegende Ende des Tubus kann abgeschrägt sein. Etwa ein bis zwei Fingerbreit darüber kann die Fluidkammer in Form eines kleinen Ballons, eines sogenannten Cuffs, angebracht sein. Dieser kann über einen am Tubus befestigten Schlauch überein Ventil mit Luft befüllt werden. Dadurch können Spalten zwischen Tubus und Trachea geschlossen werden womit die Trachea abgedichtet ist und der einzige Weg in die Lunge über den Tubus führt. Die Aerosolerzeugungsvorrichtung sowie auch die Insuffliervorrichtung werden nun ebenfalls durch den oben genannten Tubus hindurchgeführt. Ferner können noch Mittel an der Vorrichtung vorhanden sein, die eine Lagefixierung der Aerosol-erzeugungsvorrichtung, die als Düse ausgebildet sein kann, gestattet. Ein Mittel hierzu kann in einer Ausführungsform beispielsweise ein weiterer Ballon sein, der ebenfalls über einen durch den Tubus hindurchführbaren Schlauch mit Luft befüllbar ist.

In einer weiteren Ausführung wird eine Vorrichtung geeignet zur Einführung und zur Verwendung in einer schlauchförmigen Körperhöhle eines Patienten, wie z. B. in der Speiseröhre, die Vorrichtung aufweisend eine erste aufweitbare Fluidkammer zur Abdichtung eines ersten Abschnitts der Körperhöhle, eine zweite aufweitbare Fluidkammer zur Abdichtung eines zweiten Abschnitts der Körperhöhle, eine Aerosolerzeugungsvorrichtung zur Erzeugung eines therapeutischen Aerosols innerhalb eines sich zwischen dem ersten und dem zweiten Abschnitt erstreckenden Mittelabschnitts der Körperhöhle, beschrieben. Der Mittelabschnitt ist in diesem Fall auch der Behandlungsabschnitt. In dieser Ausführung sind ferner Mittel zum Insufflieren der Körperhöhle vorgesehen. Diese Mittel umfassen eine Insuffliervorrichtung mit einer Zuleitung etwa in Form eines Schlauchs, der an eine Druckpumpe anschließbar ist, welche CO₂ fördert.

Vorteilhafterweise kann die Vorrichtung noch eine Überwachungsvorrichtung, insbesondere Kamera geeignet zum Überwachen des Behandlungsabschnitts der Körperhöhle aufweisen. Die Kamera kann mit Mitteln versehen sein, die deren Drehung oder sonstige Verlagerung innerhalb des Körperhöhlen-behandlungsabschnitts erlauben.

Vorteilhafterweise kann die Vorrichtung noch eine Sensorvorrichtung geeignet zum Erfassen des in dem Behandlungsabschnitt vorhandenen Aerosols aufweisen. Die Sensorvorrichtung kann insbesondere eine IR-Diodenanordnung sein, die die Dichtigkeit des Aerosols erfasst und in elektrische Signale umwandelt. Auch die Sensorvorrichtung kann mit Mitteln versehen sein, die deren Drehung oder sonstige Verlagerung innerhalb des Körperhöhlenbehandlungsabschnitts erlauben.

Vorteilhafterweise kann die Vorrichtung noch zumindest eine Operationsvorrichtung, geeignet zu einer Manipulation der Wandung des Behandlungsabschnitts der Körperhöhle, aufweisen. Bei der Operationsvorrichtung kann es sich um ein Schneidwerkzeug, etwa eine Biopsiezange zum Entfernen von Gewebe oder um Werkzeuge zum nichtoperativen Einsatz etwa um eine Pinzette handeln.

Eine Zuleitung der Aerosolerzeugungsvorrichtung, nämlich die Druckleitung mit der gewählten Substanz oder einer Mischung verschiedener Substanzen ist durch die erste Fluidkammer hindurch in den Behandlungsabschnitt der Körperhöhle geführt.

Die Fluidkammer kann bei dieser Ausführung beispielsweise schlauchförmig ausgebildet sein, wobei der Schlauch an beiden Enden mit dafür geeigneten Mitteln, etwa Verkleben, gegenüber der Zuleitung abdichtend ausgeführt ist. Insbesondere hat es sich als Vorteil gezeigt, wenn die Aerosolvorrichtung möglichst nah an der ersten Fluidkammer und zentral zu ihr sowie auf der Längsmittelachse des Körperhöhlenbehandlungsabschnitts angeordnet ist, um den Aerosolnebel noch gleichmäßiger in dem Volumen des Behandlungsabschnitts verabreichen zu können.

In einer weiteren Ausführung ist vorgesehen, zumindest eine weitere Zuleitung durch die erste Fluidkammer hindurch in den Behandlungsabschnitt der Körperhöhle zu führen.

Insbesondere ist es von Vorteil, möglichst sämtliche verwendeten Zuleitungen, worunter sowohl Fluidleitungen als auch elektrische oder mechanische Verbindungen zu den genannten und noch zu nennenden Gegenständen verstanden werden, durch die erste Fluidkammer hindurch zu führen, insbesondere sämtliche Zuleitungen innerhalb des von der Fluidkammer gebildeten Schlauches zu führen.

In einer weiteren Ausführung der Erfindung können dazu sämtliche Zuleitungen innerhalb eines Aufnahmemittels, etwa eines Mantelschlauches, geführt sein an das sich der erste Fluidbeutel dichtend anschließt.

Ferner ist noch beschrieben, eine Intubiervorrichtung vorzusehen, wobei eine Zuleitung der Intubiervorrichtung durch die erste Fluidkammer geführt ist. Hier kann etwa bei einer Behandlung der Luftröhre und/oder der Lunge die Beatmung des Patienten auch während der mit der Vorrichtung geschaffenen medikamentösen Aerosoltherapie ohne weiteres aufrechterhalten werden.

In einer weiteren Ausführung ist ferner vorgeschlagen, dass die Länge zumindest einer Fluidkammer durch ein Verstellmittel veränderbar ist. Durch die Längenverstellung kann der Außendurchmesser der Fluidkammer besser an den Innenquerschnitt der Körperhöhle angepasst werden. Das Verstellmittel kann beispielsweise eine Teleskopvorrichtung oder ein Draht sein. Insbesondere kann auch der Abstand zwischen zwei Fluidkammern durch die Verstellmittel veränderbar sein.

Beschrieben wird ferner eine Vorrichtung zur medikamentösen Therapie eines Patienten, die Vorrichtung aufweisend ein oder zwei Fluidkammern und geeignet zu Durchführung eines Verfahrens mit den Schritten:
- Einführen der Vorrichtung in eine Körperhöhle eines Patienten,
- Einleiten eines Fluids in eine erste Fluidkammer,
- Insufflieren des Behandlungsabschnitts mit einem gasförmigen Medium,
- Einleiten eines Fluids in eine zweite Fluidkammer,
- Einleiten eines Aerosols in einen zwischen den Fluidkammern liegenden Behandlungsabschnitt der Körperhöhle mit den oben genannten Vorteilen.

Wird eine Vorrichtung mit lediglich einer Fluidkammer verwendet, entfällt naturgemäß das Einleiten eines Fluids in eine zweite Fluidkammer.

Beschrieben wird ferner eine Vorrichtung aufweisend ein Mündungsorgan, welches zumindest einen Öffnungsabschnitt der Insuffliervorrichtung und eine Düse der Aerosolerzeugungsvorrichtung aufweist, wobei das Mündungsorgan in den Behandlungsabschnitt mündet. In einer besonderen Ausführung kann dieses Mündungsorgan mit seinen Anschlüssen durch einen Tubus hindurch in den Behandlungsabschnitt geschoben werden. Der Tubus selbst weist hier an seiner äußeren Umfangsfläche die Fluidkammer in Form etwa eines Cuff auf. Die Form des Mündungsorgans wird in seinem Außendurchmesser durch die zu behandelnden Organe des Patienten sowie von den notwendigen Abmessungen des Aerosolerzeugungsvorrichtung, und sonstiger Zuleitungen, insbesondere deren Anzahl und Durchmesser beschränkt. Insbesondere kann das Mündungsorgan die Form eines flachen Zylinders aufweisen, dessen dem Behandlungsabschnitt zugewandte Stirnseite entweder plan oder konvex ausgebildet ist. Vorteilhafterweise ist das Mündungsorgan abgerundet, um eine Verletzung der zu behandelnden Organe möglichst zu vermeiden. In einer besonderen Ausführung kann das Mündungsorgan in bzw. auf einen durch die Fluidkammer hindurchgeführten Schlauch gesteckt, mit dem Schlauch verklebt, oder auf sonstige Weise fixiert werden. Ferner ist in einer alternativen Ausführung die Einstückigkeit von Schlauch und Mündungsorgan vorgesehen.

Das genannte Mündungsorgan weist ferner zumindest eine weitere Passage in den Behandlungsabschnitt auf. Diese zumindest eine Passage kann beispielsweise für Operationswerkzeuge, für Sensoren oder für die Zuführung und/oder Ableitung von Fluid, also flüssigen oder gasförmigen Medien genutzt werden. Vorteilhafterweise ist die Passage in ihrem Innendurchmesser auf einen Außendurchmesser der hindurchgeführten Gegenstände angepasst. Ferner kann noch eine druckstabile Abdichtung der Passage zum Behandlungsabschnitt hin durch Abdichtmittel, etwa durch O-Ringe vorgesehen sein.

Schließlich kann in einer weiteren Ausgestaltung die erste Fluidkammer durch einen Trokar hindurchführbar sein. In dieser Ausführung ist sichergestellt, dass das Aerosol nicht in unerwünschte Abschnitte innerhalb des Organs oder in die Umgebung gelangen kann.

Nachfolgend wird die Erfindung anhand einiger Ausführungsbeispiele näher erläutert.

Es zeigt schematisch:
Fig.1 eine erste Therapievorrichtung in perspektivischer Ansicht,
Fig. 2 bis 4 jeweils einen Schnitt durch eine Körperhöhle mit einer Aufsicht auf eine weitere Vorrichtung,
Fig. 5 und 6 jeweils eine Schnittansicht durch eine Fluidkammer,
Fig. 7 einen Schnitt durch eine Körperhöhle mit einer Aufsicht auf eine weitere Vorrichtung,
Fig. 8 und 9 je verschiedene zu einer Vorrichtung gehörende Gruppen von Gegenständen,
Fig. 10 und 11 jeweils einen Schnitt durch eine Körperhöhle mit einer Aufsicht auf eine weitere Vorrichtung,
Fig. 12 eine Vorrichtung innerhalb der Speiseröhre und des Magens,
Fig. 13 eine Vorrichtung innerhalb der Harnblase,
Fig. 14 und 15 eine nicht erfindungsgemäße Vorrichtung innerhalb der Luftröhre.

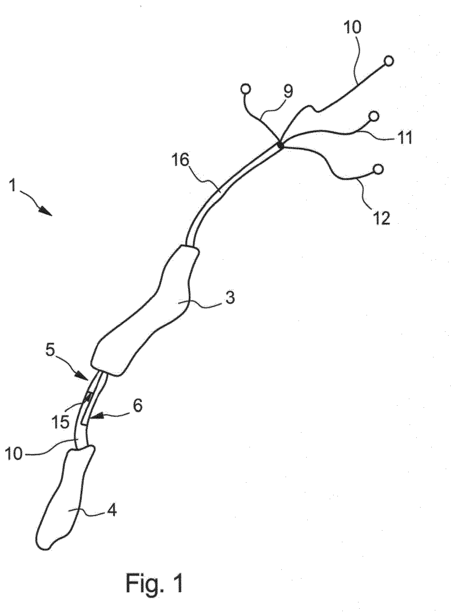

Die in Fig. 1 gezeigte Vorrichtung 1 hat eine erste aufweitbare Fluidkammer 3, eine zweite aufweitbare Fluidkammer 4, eine Aerosolerzeugungsvorrichtung 5 in Form einer an eine Zuleitung in Form einer Druckleitung 11 angeschlossenen Düse 15 mit einem Innendurchmesser von 20 Mikrometer zur Erzeugung eines therapeutischen Aerosols sowie eine Insuffliervorrichtung 6 in Form eines Schlauchs, dessen eine Öffnung etwa mittig zwischen den beiden Fluidkammern 3, 4 angeordnet ist.

Gezeigt sind insgesamt vier Zuleitungen 9-12 ; je eine Zuleitung 9, 10 für die beiden Fluidkammern zur Zuführung von Umgebungsluft unter geringem Druck (etwa 0,8 bar); der Druckleitung 11 gefüllt mit einer Substanz, einem Chemotherapeutikum zur Zuführung auf die Düse 15 mit einem Druck von etwa 20bar und der weiteren Luftzufuhrleitung 12 zur Insuffliervorrichtung zur Dehnung eines Behandlungsabschnitts der Körperhöhle, der nachfolgend gezeigt wird. Zumindest die Zuleitungen 11 und 12 sind in einem gemeinsamen Schlauchmantel 16 aufgenommen und darin durch die Fluidkammer 3 hindurchgeführt. Die Zuleitung 9 für die obere Fluidkammer 3 kann ebenfalls durch den Schlauchmantel 16 hindurchgeführt sein bzw. mit dem Schlauchmantel 16 in die Fluidkammer 3 einmünden. Alternativ kann die Fluid- bzw. Luftzufuhr für die obere Fluidkammer 3 auch unmittelbar an diese mit der Zuleitung 9 angeschlossen sein. Die gezeigte Vorrichtung und alle übrigen noch beschriebenen Vorrichtungen können für den einmaligen Gebrauch ausgelegt sein. Eine Reinigung entfällt in diesem Fall.

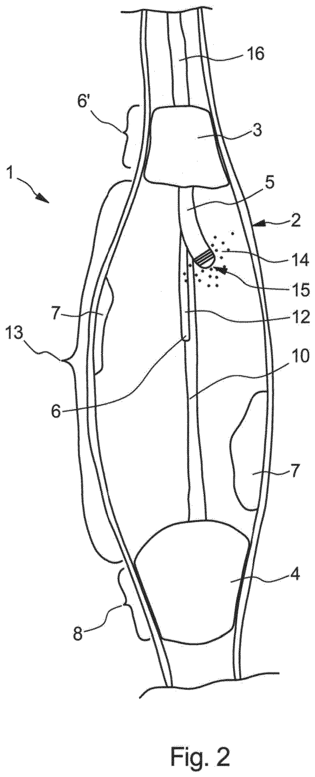

Die in Fig. 2 gezeigte Vorrichtung 1 ist in einer schlauchförmigen Körperhöhle 2 eines Patienten eingeführt gezeigt. Eine erste in einem aufgeblasenen Zustand befindliche Fluidkammer 3 dichtet einen ersten oberen Abschnitt 6'der Körperhöhle 2, hier der Luftröhre ab. Eine zweite aufweitbare Fluidkammer 4 dichtet einen zweiten unteren Abschnitt 8 der Körperhöhle 2, hier der Speiseröhre ab.

Ferner gibt eine Aerosolerzeugungsvorrichtung 5 ein therapeutische wirkendes Aerosol 14 innerhalb eines sich zwischen dem ersten und dem zweiten Abschnitt 6', 8 erstreckenden Behandlungsabschnitts 13 der Körperhöhle 2 ab. Gezeigt sind ferner noch zwei Tumore 7, die mittels des Aerosols 14 abgetötet werden.

Sämtliche Zufuhrleitungen bis auf die Zufuhrleitung für die Fluidkammer 3 sind auch hier durch die Fluidkammer 3 innerhalb eines Schlauchmantels 16 hindurchgeführt. Die Zuführleitung zur Fluidkammer 3 endet innerhalb der Fluidkammer 3.

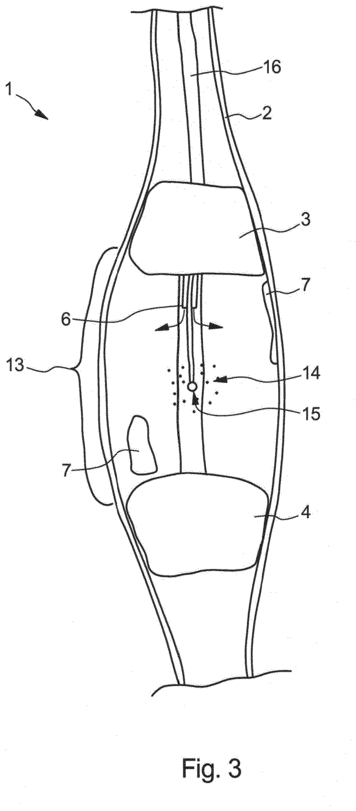

Figur 3 zeigt eine weitere Vorrichtung mit einer weiteren Anordnung der Aerosolerzeugungsvorrichtung 5 mit Düse 15 und der Insuffliervorrichtung 6.

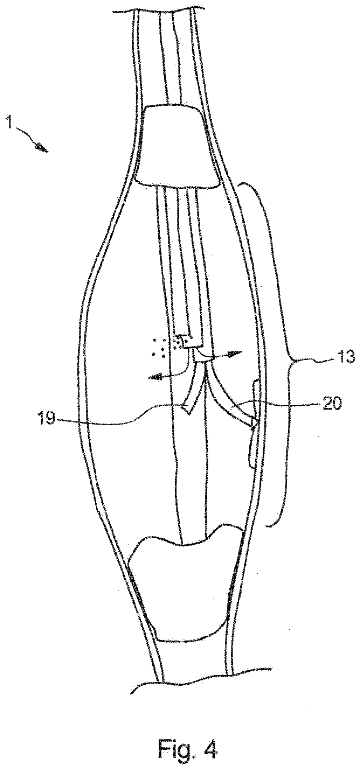

In Figur 4 zeigt die Vorrichtung 1 noch eine Überwachungsvorrichtung 19, insbesondere eine Kamera, die zwischen der ersten und der zweiten Fluidkammer angeordnet ist, geeignet zum Überwachen des Behandlungsabschnitts der Körperhöhle sowie eine Operationsvorrichtung 20, in Form einer Schneidanodenanordnung geeignet zu einer Manipulation der Wandung des Behandlungsabschnitts der Körperhöhle.

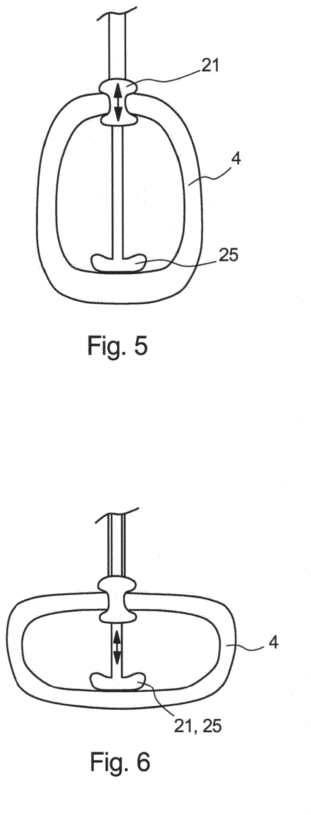

Die Figuren 5 und 6 zeigen schematisch noch wie die Länge zumindest einer Fluidkammer 4 durch ein Verstellmittel 21 in Fig. 6 in Form einer Teleskopvorrichtung mit einem auf die Fluidkammer einwirkenden Stempel 25 veränderbar ist.

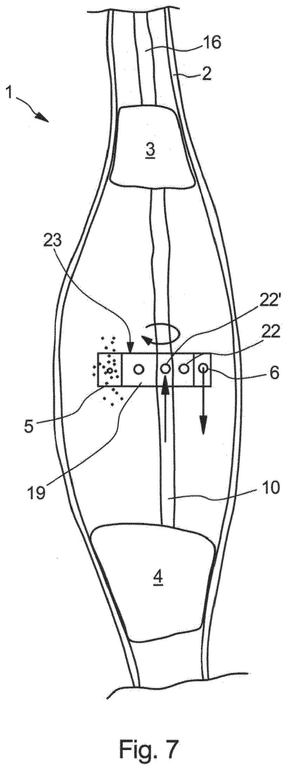

In Fig. 7 ist ein erweitere Anordnung einer alternativen Vorrichtung schematisch gezeigt. Insbesondere sind die Aerosolerzeugungsvorrichtung 5, die Kamera 19, ein Sensor 22, die Insuffliervorrichtung 6 und eine Absaugeinrichtung 22' innerhalb eines Drehorgans 23 angeordnet, dass sich unabhängig von der Position der Fluidkammern 3, 4 verdrehen lässt.

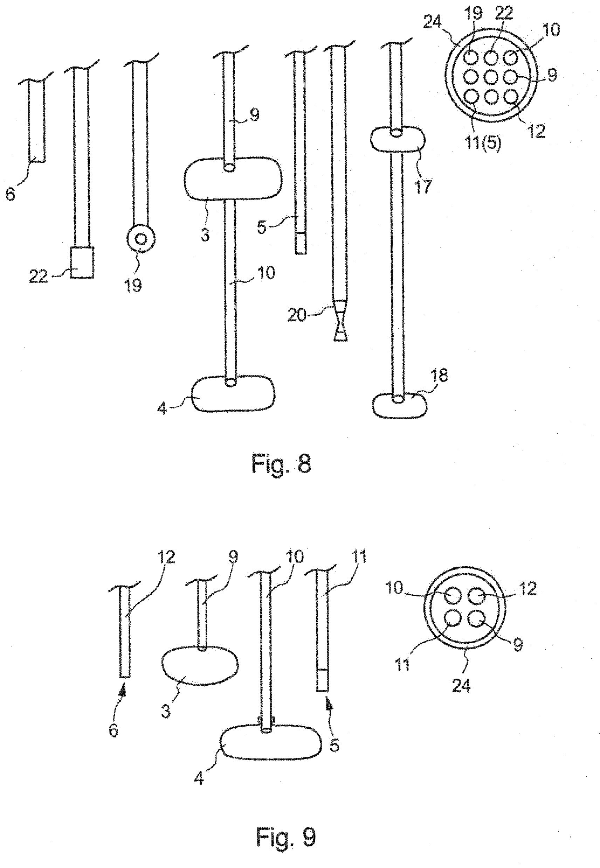

In Figur 8 sind Teile einer Vorrichtung, nämlich die Aerosolerzeugungsvorrichtung 5, die Insuffliervorrichtung 6, die Überwachungsvorrichtung 19, die Sensorvorrichtung 22, vier Fluidkammern 3, 4, 17 und 18, eine Operationsvorrichtung 20 mit ihren jeweiligen Zuführleitungen schematisch gezeigt. Insbesondere ist die gemeinsame Anordnung der Leitungen zu den genannten Elementen innerhalb eines noch vor der Fluidkammer 3 angeordneten Aufnahmeschlauches 24 in einer Schnittansicht gezeigt.

Figur 9 zeigt die Teile der Vorrichtung von Fig. 1, nämlich die Aerosolerzeugungsvorrichtung 5 , die Insuffliervorrichtung6 und zwei Fluidkammern 3, 4.

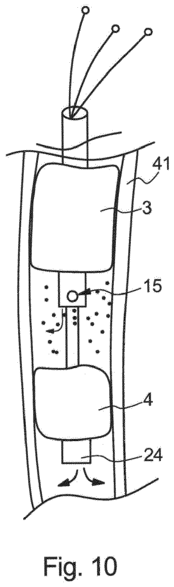

Fig. 10 zeigt eine Vorrichtung zum Einsatz in einer weiteren Körperhöhle nämlich der Luftröhre 41. Zu erkennen ist die Durchführung eines Intubiermittels, hier eines Schlauches 24 zur Intubation des Patienten durch beide Fluidkammern 3, 4.

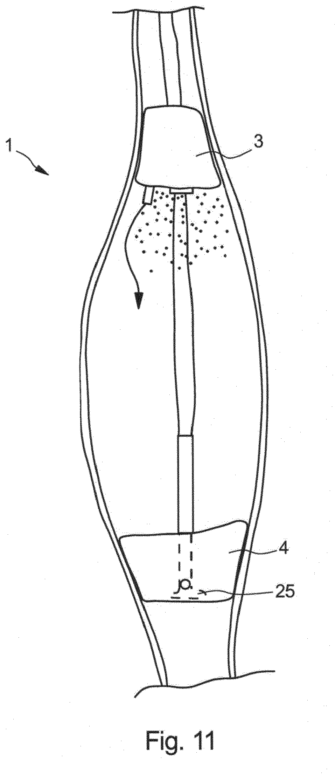

In Fig. 11 ist eine Vorrichtung 1 mit einer verstellbaren Fluidkammer 4 gezeigt, die mit einem Verstellmittel in Form eines Stempels 25 entsprechend Fig. 6 verstellbar ist.

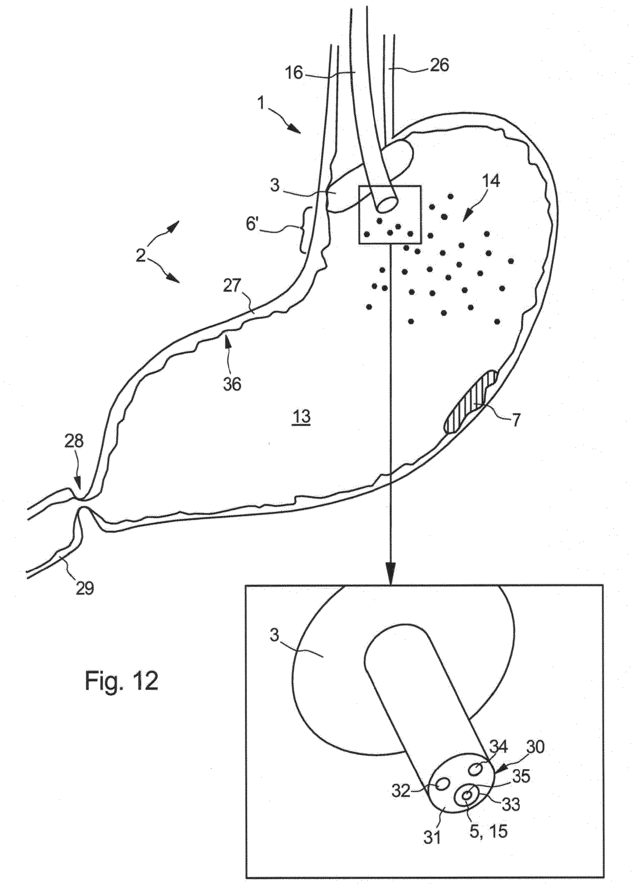

Die Figur 12 zeigt eine Vorrichtung 1, angeordnet innerhalb einer Körperhöhle 2 in Form der Speiseröhre 26 und des Magens 27. Gezeigt ist ferner noch ein pathologischer Bereich hier in Form eines Tumors 7 innerhalb des Magens 27.Die Vorrichtung 1 umfasst eine Fluidkammer 3 und ein durch die Fluidkammer hindurchgeführten Schlauch 16. Die Fluidkammer 3 in Form eines Ballons dichtet den Magen 27 zur Speiseröhre 26 hin in einem Körperhöhlenabschnitt 6' ab. Der Behandlungsabschnitt 13 ist hier durch das vom Magen 27 aufgespannte insufflierte Volumen gebildet und wird vom Pylorus 28 zum Darm 29 hin begrenzt. Der genannte Schlauch 16 endet in dem Behandlungsabschnitt 13 mit einem Mündungsorgan 30 mit plan ausgebildeter Stirnseite 31. Das Mündungsorgan 30 ist in einer vergrößerten Ansicht dargestellt. Der Außendurchmesser des Mündungsorgans beträgt etwa 10mm. Ein anderer Durchmesser kann je nach Größe der Organe ebenfalls vorgesehen sein. Üblicherweise ist der Durchmesser für ein Mündungsorgan, das innerhalb des Magens angewandt wird, nicht größer als 13mm. Auf der Stirnseite 31 des Mündungsorgans sind drei Öffnungen 32 bis 34 ausgebildet. Eine Öffnung 32 ist für die Einleitung eines gasförmigen Mediums, nämlich CO₂ vorgesehen, und ist Bestandteil der Insuffliervorrichtung 6. Eine zweite Öffnung 33 weist die Aerosolerzeugungsvorrichtung 5 in Form einer Düse 15 mit einem Düseninnendurchmesser von 20 Mikrometer auf. Die dritte Öffnung 34 ist optional und bildet das Ende einer Passage zur Durchführung beispielsweise eines Operationswerkzeugs, eines Sensors 22 oder einer Fluidzufuhrleitung oder einer Fluidableitung. Nicht näher gezeigt ist der Anschluss von zwei Fluidleitungen an das Mündungsorgan. Insbesondere bildet das Mündungsorgan 30 eine runde Durchgangsöffnung auf die gegenüber der Stirnseite in einem Anschlussstutzen endet, an den ein Insufflierschlauch angeschlossen wird. Für die Düse 15 weist das Mündungsorgan 30 stirnseitig eine ebenfalls nicht gezeigte Ausnehmung mit Innengewinde auf, mit dem ein Außengewinde der Düse 15 verschraubar ist. Gegenüber der Stirnseite 31 ist ebenfalls eine Schraubverbindung vorgesehen, mit der ein Druckschlauch an die Düse 15 angeschlossen werden kann.

Während der Behandlung des Behandlungsbereichs 13 ist der Druckschlauch unter hohem Druck, beispielsweise mit 20 bar, mit zumindest einem in flüssiger Form vorliegenden Chemotherapeutikum gefüllt. Die Flüssigkeit wird dann bei Austritt aus der kleinen Düsenöffnung 35 schlagartig druckentlastet und infolgedessen innerhalb des zuvor durch die Insuffliervorrichtung mit CO₂ gedehnten Magens zu einem Nebel, dem Aerosol 14 zerstäubt. Es hat sich überraschend gezeigt, dass das Aerosol 14 äußerst gleichmäßig innerhalb des Magenvolumens verteilt werden kann und die Flüssigkeit in Form des Chemotherapeutikums entsprechend gleichmäßig auf die innere Oberfläche 36 des durch das Insufflieren aufgeweiteten Magens einwirken kann. Optional kann, etwa durch Verwendung von entsprechenden Potentialelektroden, das Aerosol 14 auch elektrisch positiv geladen sein und der Körper des Patienten demgegenüber negatives Potential aufweisen. Hierbei wird erreicht, dass das Aerosol 14 sich mit dem durch den Potentialunterschied gebildeten Gradientenfeld noch gleichmäßiger an den Mageninnenwänden 36 verteilt.

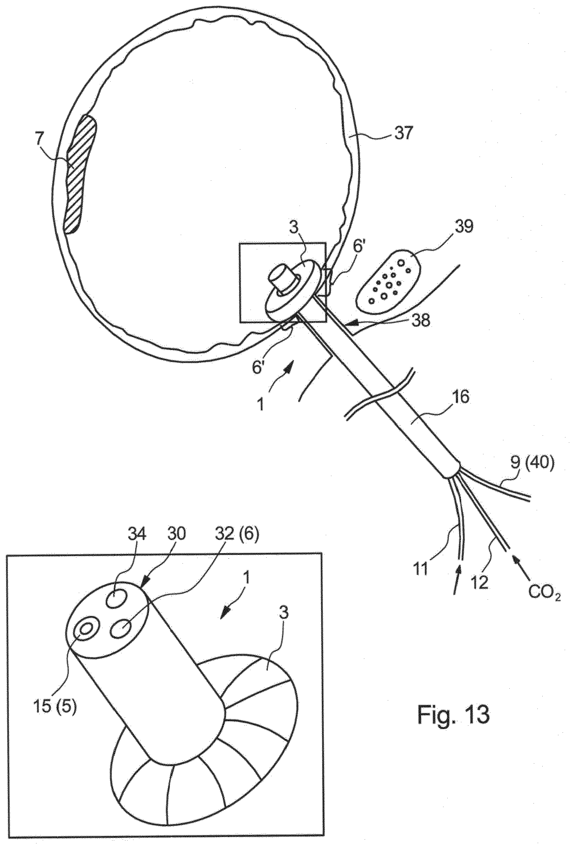

Fig. 13 zeigt eine Vorrichtung 1 innerhalb einer Körperhöhle 2, gebildet aus Harnblase 37 und Urethra 38 neben dem Os pubis 39 in einer schematisierten Schnittdarstellung. Dargestellt ist der Verschluss der Harnblase 37 in einem Körperhöhlenabschnitt 6' innerhalb der Harnblase 37 gegenüber der Urethra 38 mittels einer aufgeblasenen Fluidkammer 3 in Form eines mit Luft befüllten Ballons. Wie in Figur 12 wird auch hier ein Mündungsorgan 30 verwendet, dass eine Düse 15 für die Einleitung eines über eine Hochdruckleitung 11 anliegenden Chemotherapeutikums, ferner eine optionale Öffnung 34 eines Arbeitskanals und eine Öffnung 32 für die Zuleitung 12 der Fluidzufuhr von CO₂ in die Harnblase 37 für deren Insufflation aufweist. Innerhalb des durch die Fluidkammer 3 geführten Mantelschlauchs 16, der mit dem Mündungsorgan 30 abdichtet, ist die Hochdruckleitung 11 und die CO₂-Leitung 12 zur Insufflation geführt. Ein Luftschlauch 40 zum Befüllen des Ballons kann wie gezeigt entweder ebenfalls als Zuleitung 9 innerhalb des Mantelschlauchs 16 geführt sein oder aber außerhalb davon unmittelbar mit der Fluidkammer 3 verbunden werden. In der gezeigten Vergrößerung eines Abschnitts der Figur 13 mündet ferner noch ein Arbeitskanal mit einer Öffnung 34 auf der Stirnseite 31 des Mündungsorgans 30. Dieser Arbeitskanal kann auch fortgelassen werden oder es können noch ein oder mehrere weitere Arbeitskanäle bzw. Passagen stirnseitig in dem Mündungsorgan angeordnet sei. Das Mündungsorgan 30 kann einen Außendurchmesser von maximal 10mm erreichen. Geringere Durchmesser von 5-7mm sind üblich bei einer Verwendung der Vorrichtung innerhalb der Harnröhre 38.

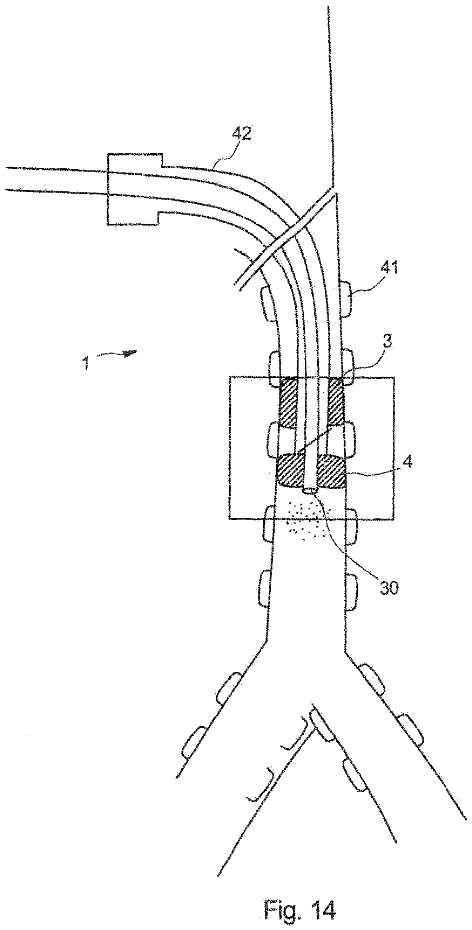

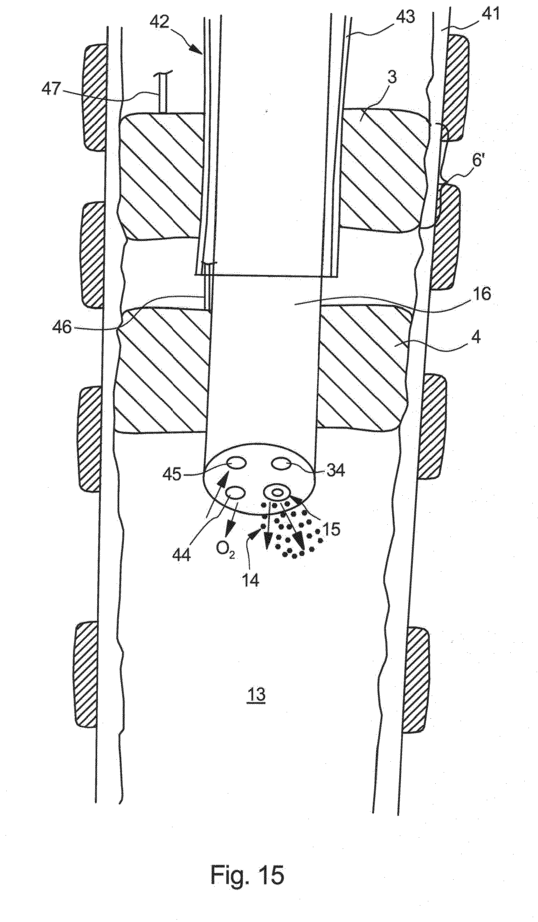

Fig. 14 und Fig. 15 zeigen schematisch eine nicht erfindungsgemäße Vorrichtung 1, angeordnet innerhalb einer Körperhöhle in Form der Luftröhre 41. Die Vorrichtung beinhaltet hier einen Endotrachealtubus 42 mit einer ersten Fluidkammer 3 in Form eines kleinen Ballons und einem Schlauch 43 durch den üblicherweise beatmet wird. Hier allerdings erfolgt neben der Beatmung mittels Gaszufuhr 44 mit Sauerstoff oder einem Gasgemisch und Gasabfuhr 45 auch erfindungsgemäß die Verabreichung von Aerosol 14 mittels der Düse 15 sowie optional die Luftzufuhr 46 für eine weitere Fluidkammer 4 und ebenfalls optional noch die Durchführung weiterer optionaler Mittel durch eine Öffnung 34 eines Arbeitskanals in den Behandlungsabschnitt 13.

Die Luftzuführung 47 der ersten Fluidkammer 3 erfolgt üblicherweise außerhalb des gezeigten Tubusschlauches 43. Die Luftzuführung 46 zu der weiteren Fluidkammer 4 kann ebenfalls außerhalb des Tubus und außerhalb des durch den Tubusschlauch geführten Mantelschlauchs 16 erfolgen.

Es versteht sich, dass die Zuleitung zur Fluidkammer 4 auch außerhalb zu dem durch die Fluidkammer 3 geführten Mantelschlauch geführt sein kann. Ferner versteht es sich, dass hinsichtlich der Gasabfuhr 45 die mit Aerosol 14 angereichert sein kann, das hier nicht gezeigte Beatmungsgerät, das an die Gasabfuhr 45 angeschlossen ist, entsprechende Rückhaltemittel, etwa Filter oder Kondensationsvorrichtungen bereitstellen sollte, um die Kontamination der Umgebung außerhalb des Behandlungsabschnitts 13, hier der Luftröhre 41 und der Lunge zu verhindern. Im Übrigen kann in einer weiteren Ausführung die zweite Fluidkammer 4 fortgelassen werden, sofern sichergestellt ist, dass kein Gas den ersten von der ersten Fluidkammer 3 bedeckten Abschnitt 6' passieren kann.

### LISTE DER BEZUGSZEICHEN

1 Vorrichtung
2 Körperhöhle
3 Fluidkammer
4 Fluidkammer
5 Aerosolerzeugungsvorrichtung
6 Insuffliervorrichtung,
6' Abschnitt
7 Tumor
8 Abschnitt
9 Zuleitung
10 Zuleitung
11 Zuleitung, Druckleitung
12 Zuleitung, Luftzufuhrleitung
13 Behandlungsabschnitt
14 Aerosol
15 Düse
16 Schlauchmantel
17 Fluidkammer
18 Fluidkammer
19 Überwachungsvorrichtung
20 Operationsvorrichtung
21 Verstellmittel
22 Sensor
22' Absaugeinrichtung
23 Drehorgan
24 Aufnahmeschlauch
25 Stempel
26 Speiseröhre
27 Magen
28 Pylorus
29 Darm
30 Mündungsorgan
31 Stirnseite
32 Öffnung CO₂
33 Öffnung
34 Öffnung optional
35 Düsenöffnung
36 Oberfläche, Mageninnenwände
37 Harnblase
38 Urethra
39 Os pubis
40 Luftschlauch
41 Luftröhre
42 Endotrachealtubus
43 Schlauch
44 Gaszufuhr, O₂
45 Gasabfuhr
46 Luftzufuhr
47 Luftzuführung.

## Patentansprüche

1. Vorrichtung (1), die konfiguriert ist:
zur Einführung in eine Körperhöhle (2) eines Patienten, insbesondere in ein Organ (26, 27, 37) des Patienten;
zur Insufflation eines gasförmigen Mediums zum Aufweiten der Körperhöhle (2), wobei das gasförmige Medium CO₂ ist; und
zum Erzeugen eines therapeutischen Aerosols innerhalb der Körperhöhle (2), die Vorrichtung (1) aufweisend:
- eine erste aufweitbare Fluidkammer (3), konfiguriert zur Abdichtung eines ersten Abschnitts (6') der Körperhöhle (2);
- eine Aerosolerzeugungsvorrichtung (5), konfiguriert zur Erzeugung eines therapeutischen Aerosols aus einer Substanz in einem sich nach dem ersten Abschnitt (6') erstreckenden Behandlungsabschnitt (13) der Körperhöhle (2), wobei die Aerosolerzeugungsvorrichtung (5) eine zur Zuführung der Substanz konfigurierte erste Zuleitung umfasst; und
- eine Insuffliervorrichtung (6), konfiguriert zum Insufflieren des gasförmigen Mediums in den Behandlungsabschnitt (13) der Körperhöhle (2), wobei die Insuffliervorrichtung (6) eine zur Zuführung des gasförmigen Mediums konfigurierte zweite Zuleitung sowie Mittel konfiguriert zur Druckbeaufschlagung des gasförmigen Mediums umfasst;
wobei die Vorrichtung derart strukturiert ist, dass:
nachdem die erste Fluidkammer (3) aufgeweitet worden ist, womit die aufgeweitete erste Fluidkammer (3) dicht an einer Wandung der Körperhöhle (2) anliegt, so dass der erste Abschnitt (6') der Körperhöhle (2) von der aufgeweiteten ersten Fluidkammer (3) abgedichtet ist, die erste Zuleitung und die zweite Zuleitung durch die erste Fluidkammer (3) hindurch in den Behandlungsabschnitt (13) der Körperhöhle (2) geführt werden können, sodass dann das gasförmige Medium durch die zweite Zuleitung in den Behandlungsabschnitt (13) der Körperhöhle insuffliert werden kann, und danach das therapeutische Aerosol (14) in dem mit dem gasförmigen Medium insufflierten Behandlungsabschnitt (13) der Körperhöhle (2) erzeugt werden kann.

2. Vorrichtung nach Anspruch 1,
die Vorrichtung ferner aufweisend eine zweite aufweitbare Fluidkammer (4) zur Abdichtung eines zweiten Abschnitts (8) der Körperhöhle, wobei der Behandlungsabschnitt (13) als Mittelabschnitt zwischen dem ersten und dem zweiten Abschnitt (6', 8) vorgesehen ist.

3. Vorrichtung nach Anspruch 1 oder 2, ferner aufweisend zumindest einen Gegenstand aus der Gruppe:
- Überwachungsvorrichtung (19), insbesondere eine Kamera, geeignet zum Überwachen des Behandlungsabschnitts (13) der Körperhöhle (2),
- Sensorvorrichtung (22), geeignet zum Erfassen des in dem Behandlungsabschnitt (13) vorhandenen Aerosols (14),
- Operationsvorrichtung (20), geeignet zu einer Manipulation der Wandung (36) des Behandlungsabschnitts (13) der Körperhöhle (2).

4. Vorrichtung (1) nach einem der vorangegangenen Ansprüche,
wobei mittels der Insuffliervorrichtung (6) ein Druck zwischen 5 mbar und 30 mbar, bevorzugt zwischen 10 mbar und 15 mbar innerhalb des Behandlungsabschnitts (13) erzeugbar ist.

5. Vorrichtung (1) nach einem der vorangegangenen Ansprüche,
wobei die Länge zumindest der oder einer der Fluidkammer (3, 4) durch ein Verstellmittel (21) veränderbar ist.

6. Vorrichtung (1) nach Anspruch 2, wobei der Abstand zwischen der ersten und der zweiten Fluidkammer (3, 4) mittels einer Teleskopvorrichtung veränderbar ist.

7. Vorrichtung (1) nach einem der Ansprüche 1 bis 6,
ferner aufweisend ein Mündungsorgan (30), welches zumindest einen Öffnungsquerschnitt (32, 44) der Insuffliervorrichtung (6) und eine Düse (15) der Aerosolerzeugungsvorrichtung (5) aufweist, wobei das Mündungsorgan (30) in den Behändlungsabschnitt (13) mündet.

8. Vorrichtung (1) nach Anspruch 7,
wobei das Mündungsorgan (30) ferner zumindest eine weitere Passage (34, 45) in den Behandlungsabschnitt (13) aufweist.

9. Vorrichtung (1) nach einem der vorangegangenen Ansprüche, wobei die erste Fluidkammer (3) durch einen Trokar (42) hindurchführbar ist.

## Claims

1. A device (1) configured:
for insertion into a body cavity (2) of a patient, in particular into an organ (26, 27, 37) of the patient;
for insufflation of a gaseous medium to expand the body cavity (2), wherein the gaseous medium is CO₂; and
for generating a therapeutic aerosol within the body cavity (2),
the device (1) comprising:
- a first expandable fluid chamber (3) configured to seal a first section (6') of the body cavity (2);
- an aerosol generating device (5) configured to generate a therapeutic aerosol from a substance in a treatment section (13) of the body cavity (2) extending after the first section (6'), the aerosol generating device (5) including a first supply line configured to supply the substance; and
- an insufflation device (6) configured to insufflate the gaseous medium into the treatment section (13) of the body cavity (2), the insufflation device (5) comprising a second supply configured to supply the gaseous medium and means configured to pressurise the gaseous medium;
the device (1) being structured such that:
after the first fluid chamber has been expanded, whereby the expanded first fluid chamber (3) closely abuts a wall of the body cavity (2) so that the first section (6') of the body cavity (2) is sealed by the expanded first fluid chamber (3), the first supply line and the second supply line can be passed through the first fluid chamber (3) into the treatment portion (13) of the body cavity (2), so that the gaseous medium can then be insufflated through the second supply line into the treatment section (13) of the body cavity (22), and thereafter the therapeutic aerosol (14) can be generated in the treatment section (13) of the body cavity (2) insufflated with the gaseous medium.

2. The device (1) according to claim 1, further including a second expandable fluid chamber (4) for sealing a second section (8) of the body cavity (2), wherein the treatment section (13) is provided as a central section between the first and the second section (6', 8).

3. The device (1) according to claim 1 or 2, further including at least one item selected from the group of:
- monitoring means (19) suitable for monitoring the treatment section (13) of the body cavity (2), wherein the monitoring means includes a camera,
- sensor means (22) suitable for detecting the aerosol (14) present in the treatment section (13), and
- operation apparatus (20) suitable for manipulation of the wall (36) of the treatment section (13) of the body cavity (2).

4. The device (1) according to one of the preceding claims, wherein a pressure between about 5 mbar and about 30 mbar, and preferably between about 10 mbar and about 15 mbar, is generated with the intubation apparatus (6).

5. The device (1) according to one of the preceding claims, wherein the length of at least one of the fluid chambers (3, 4) is adjustable using adjustment means (21).

6. The device (1) according to claim 2, wherein the distance between two fluid chambers (3, 4) is changeable using a telescopic device.

7. The device (1) according to one of the claims 1 bis 6, further including an outlet means (30) including at least an opening cross-section (32, 44) of the insufflation apparatus (6) and a nozzle (15) of the aerosol generating apparatus (5), wherein the outlet means (30) opens into the treatment section (13).

8. The device (1) according to claim 7, wherein the outlet means (30) further includes at least one further passage (34, 45) leading into the treatment section (13).

9. The device (1) according to one of the preceding claims, wherein the first fluid chamber (3) is feedable through a trocar (42).

## Revendications

1. Dispositif (1) qui est configuré :
pour une introduction dans une cavité corporelle (2) d'un patient, en particulier dans un organe (26, 27, 37) du patient,
pour l'insufflation d'un fluide gazeux, permettant l'élargissement de la cavité corporelle (2), le fluide gazeux étant du CO₂; et
pour la production d'un aérosol thérapeutique (14) à l'intérieur de la cavité corporelle (2), le dispositif présentant :
- une première chambre de fluide (3) expansible, configurée pour l'obturation d'une première partie (6') de la cavité corporelle (2);
- un dispositif de production d'aérosol (5), configuré pour la production d'un aérosol thérapeutique à partir d'une substance dans une partie de traitement (13) de la cavité corporelle (2), s'étendant après la première partie (6'), le dispositif de production d'aérosol (5) comprenant une première alimentation configurée pour l'apport de la substance; et
- un dispositif d'insufflation (6) configuré pour l'insufflation du fluide gazeux dans la partie de traitement (13) de la cavité corporelle (2), le dispositif d'insufflation (6) comprenant une deuxième alimentation configurée pour l'apport du fluide gazeux, ainsi qu'un moyen configuré pour la pressurisation du fluide gazeux,
le dispositif étant structuré de telle sorte que:
après l'expansion de la première chambre de fluide (3), permettant un appui étanche de la première chambre de fluide (3) expansée contre une paroi de la cavité corporelle (2) de telle sorte que la première partie de la cavité corporelle (2) soit obturée par la première chambre de fluide expansée (3),
la première alimentation et la deuxième alimentation puissent être guidées dans la partie de traitement (13) de la cavité corporelle (2) à travers la première chambre de fluide (3) afin que le fluide gazeux puisse être insufflé dans la partie de traitement (13) de la cavité corporelle (2) en passant par la deuxième alimentation, puis que l'aérosol thérapeutique (14) puisse être produit dans la partie de traitement (13) de la cavité corporelle (2), dans laquelle le fluide gazeux est insufflé.

2. Dispositif (1) conformément à la revendication 1, le dispositif présentant en outre une deuxième chambre de fluide (4) expansible pour l'obturation d'une deuxième partie (8) de la cavité corporelle, la partie de traitement (13) étant prévue comme partie centrale entre la première et la deuxième partie (6', 8).

3. Dispositif (1) conformément à la revendication 1 ou 2, présentant en outre au moins un objet du groupe :
- dispositif de contrôle (19), en particulier une caméra, approprié au contrôle de la partie de traitement (13) de la cavité corporelle (2),
- dispositif capteur (22), approprié à la détection de l'aérosol (14) présent dans la partie de traitement (13),
- dispositif d'opération (20), approprié à une manipulation de la paroi (36) de la partie de traitement (13) de la cavité corporelle (2).

4. Dispositif (1) conformément à l'une des revendications précédentes,
une pression entre 5 mbars et 30 mbars, préférablement entre 10 mbars et 15 mbars, pouvant être générée à l'intérieur de la partie de traitement (13) au moyen du dispositif d'insufflation (6).

5. Dispositif (1) conformément à l'une des revendications précédentes, la longueur au moins de la ou de l'une des chambres de fluide (3) pouvant être modifiée par un moyen de réglage (21).

6. Dispositif (1) conformément à la revendication 2, la distance entre la première et la deuxième chambre de fluide (3,4) pouvant être modifiée au moyen d'un dispositif télescopique.

7. Dispositif (1) conformément à l'une des revendications 1 à 6, présentant en outre un organe d'entrée (30) qui présente au moins une section d'ouverture (32, 44) du dispositif d'insufflation (6) et une buse (15) du dispositif de production d'aérosol (5), l'organe d'entrée (30) aboutissant dans la partie de traitement (13).

8. Dispositif (1) conformément à la revendication 7,
l'organe d'entrée (30) présentant en outre au moins un autre passage (34, 45) dans la partie de traitement.

9. Dispositif (1) conformément à l'une des revendications précédentes,
la première chambre de fluide (3) pouvant être traversée par un trocart (42).
